# EUROPEAN PATENT APPLICATION

(11) **EP 1 618 883 A1**
(43) Date of publication of application: **25.01.2006**
(21) Application number: 04728923.6
(22) Date of filing: 22.04.2004
(51) Int. Cl.: A61K 31/734, A61K 33/44, A61K 9/06, A61P 1/10

(54) **AGENT FOR PREVENTING AND AMELIORATING CONSTIPATION**

(30) Priority: 25.04.2003 JP 2003122470
(71) Applicant: Kouki Bussan Yugenkaisha, Tokyo 168 (JP)
(72) Inventor: AOYAGI, Juuro, Mitaka-shi, Tokyo 1810001 (JP)
(74) Representative: Köster, Hajo
(86) International application number: PCT/JP2004/005761
(87) International publication number: WO 2004/096243

(57) **Abstract**

A novel agent for preventing and healing constipation which heals the symptom of constipation very effectively and entails absolutely no side effect is provided. This agent for preventing and healing constipation is formed by containing a hydrogen of a polyvalent metal salt of alginic acid possessing a spherical or ovaloid shape having a short diameter in the range of 1 - 10 mm and an aspect ratio (long diemter/short diameter) in the range of 1 - 25. The hydrogel of a polyvalent metal salt of alginic acid is obtained by dropping an aqueous solution of sodium alginate into an aqueous solution of a polyvlant metal salt. It is optionally stored in water and/or a hydrogen compound of a natural compound possessing a free acid group capable of reacting and sequestering a basic compound.

## Description

### Technical Field

This invention relates to an agent for preventing and healing constipation. More particularly, this invention relates to an agent for preventing and healing constipation, characterized by containing a spherical or ovaloid hydrogel of a polyvalent metal salt of alginic acid.

### Background Art

The constipation is the symptom in which the feces persists for a long time in the bowel, absorbs water, and compels the patient to experience difficulty in discharging it from the bowel. The constipation is induced by the fact that the series of defecatingmechanisms are impeded by various causes. It is said that normal persons defecate at least three or more times per week. Generally, the cases of experiencing absence of motion over a period of three to four days are treated as the symptoms of constipation. Particularly, the cases of constipation complicated by such symptoms as abdominalgia, abdominal distention, lumbago, headache, and anorexia are in need of therapy. For the therapy of the constipation, salts, saccharides, distensible, infiltrating, or mucilaginous purgatives, suppository, mechanical purgative such as enemata, intestinal stimulating or colic stimulating purgative, parasympathetic nerve stimulating agent, and herbal medicine are being used at present.

The present inventors formerly developed an adsorbent formed by dispersing activated carbon in such a physiologically allowable gel-like dispersing medium as calcium alginate (refer, for example, to the official gazette of JP-A HEI 9-75723 and U.S. Patent No. 6,299,867). This adsorbent, by oral administration, is enabled to adsorb and remove a harmful substance present in the alimentary system. The fact that the hydrogel of a polyvalent metal salt of alginic acid possessing specific size and shape or the hydrogel incorporating activated carbon thereon and utilized for this invention heals the symptom of constipation, however, has not been known.

This invention has been initiated in view of the aforementioned problems encountered by the conventional technique and has for an object thereof the provision of a novel agent for preventing and healing constipation which heals the symptom of constipation very effectively and entails absolutely no secondary effect.

### Disclosure of the Invention

The present inventors have pursued a diligent study in search of an agent for preventing and healing constipation. They have consequently found that the hydrogel of a polyvalent metal salt of alginic acid possessing specific shape and size dramatically heals or prevents the symptom of constipation. This invention has been perfected as a result.

Specifically, this invention concerns an agent for preventing and healing constipation which contains the hydrogel of a polyvalent metal salt of alginic acid possessing a spherical or ovaloid shape having a short diameter in the range of 1 - 10 mm and an aspect ratio (long diameter/short diameter) in the range of 1 - 2.5.

This invention also concerns the aforementioned agent for preventing and healing constipation, wherein the aforementioned hydrogel of a polyvalent metal salt of alginic acid is obtained by dropping an aqueous solution of sodium alginate to an aqueous solution of a polyvalent metal salt.

This invention also concerns the aforementioned agent for preventing and healing constipation, which is obtained by having preserved in a hydrogel compound water and/or a natural compound possessing a free acid radical capable of reacting with and complementing a basic compound.

This invention further concerns an agent for preventing and healing constipation, wherein the aforementioned natural compound has a molecular weight of not less than 10,000.

This invention also concerns an agent for preventing and healing constipation, wherein the aforementioned acid radical is a carboxyl group or a sulfuric acid group.

This invention further concerns an agent for preventing and healing constipation, wherein the aforementioned natural compound is at least one member selected from the group consisting of alginic acid, fumaric acid, pectin, polydextrose, chondroitin sulfuric acid, and carrageenin.

This invention concerns an agent for preventing and healing constipation, wherein the hydrogel of the aforementioned natural compound contains an activated carbon in an amount in the range of 1 - 90 mass%.

This invention also concerns an agent for preventing and healing constipation, wherein the volume ratio of the aforementioned hydrogel of a polyvalent metal salt of alginic acid/water or the aforementioned natural compound is in the range of 2 : 1 - 1 : 2.

This invention concerns a method for the production of an agent for preventing and healing constipation containing the hydrogel of a polyvalent metal salt of alginic acid, which comprises dropping an aqueous sodium alginate solution to an aqueous polyvalent metal salt solution and subsequently washing the resultant solution with water.

This invention concerns the aforementioned method, wherein the concentration of the aqueous sodium alginate solution is in the rnge of 0.002 - 5 mass% and the concentration of the polyvalent metal salt is in the range of 1 - 10 mass%.

This invention further concerns the aforementioned method, wherein the amount of the aqueous sodium alginate solution to be dropped is in the range of 0.1 - 5 ml/drop.

This invention further concerns the aforementioned method, wherein the aforementioned aqueous sodium alginate solution is dropped through a nozzle having the inner diameter of the leading terminal part in the range of 0.1 - 5 mm. Best Mode for Carrying out the Invention

The agent for preventing and healing constipation contemplated by this invention is formed by containing the hydrogel of a polyvalent metal salt of alginic acid possessing a spherical or ovaloid shape having a short diameter in the range of 1 - 10 mm and an aspect ratio (long diameter/short diameter) in the range of 1 - 2.5.

The polyvalent metal salts of alginic acid which are usable in this invention include calcium salts, magnesium salts, iron salts, zinc salts, copper salts, selenium salts, chromium salts, and manganese salts of alginic acid, for example. Among other polyvalent metal salts enumerated above, calcium alginate is used particularly advantageously.

The hydrogel of a polyvalent metal salt of alginic acid to be used in this invention possesses a spherical or ovaloid shape having a short diameter in the range of 1 - 10 mm and preferably in the range of 3 - 7 mm and an aspect ratio (long diameter/short diameter) in the range of 1 - 2.5 and preferably in the range of 1 - 1.5. If the short diameter of the hydrogel of a polyvalent metal salt of alginic acid falls short of 1 mm, the shortage will result in decreasing the dynamic effect of the hydrogel in the feces and preventing the effect of promoting defecation from being manifested fully satisfactorily. Conversely, if the short diameter of the hydrogel of a polyvalent metal salt of alginic acid exceeds 10 mm or if the aspect ratio thereof exceeds 2.5, the overage will be at a disadvantage in preventing the hydrogel from easily moving around in the feces and similarly preventing the effect of promoting defecation from being satisfactorily acquired.

In this invention, the hydrogel of a polyvalent metal salt of alginic acid possessing a degree of crosslinking in the range of 30 - 90% and preferably in the range of 40 - 80% is ideally used. If the degree of crosslinking of the hydrogel of a polyvalent metal salt of alginic acid falls short of 30%, the shortage will result in decreasing the dynamic effect of the hydrogel in the feces and preventing the effect of promoting defecation from being obtained satisfactorily because the gel acquires a large water content, fails to retain the shape thereof intact, and deforms. Conversely, if the degree of crosslinking of the hydrogel of a polyvalent metal salt of alginic acid exceeds 90%, the overage will be at a disadvantage in suffering the functional group (-COOH or -COONa) in the gel to decrease and preventing the state of hydration from being maintained easily.

The term "degree of crosslinking" as used herein refers to the state in which the functional group in the gel is cross-linked by a polyvalent metal. This degree of crosslinking is obtained by determining the amount of the residual functional group by such a method as, for example, infrared absorption spectrum, quantitative determination of metal, chelatometry, gravimetric analysis, or elemental analysis (such as Journal of Industrial Chemistry, Vol. 73, No. 12, pages 2688 - 2691 (1970), published by Japan Chemical Society).

The hydrogel of a polyvalent metal salt of alginic acid to be used in this invention can be produced by dropping an aqueous solution of sodium alginate into an aqueous solution of a polyvalent metal salt and subsequently washing the resultant solution with water. In this case, the concentration of the aqueous solution of sodium alginate to be dropped is in the range of 0.002 - 5 mass% and preferably in the range of 0.1 - 2 mass% and the amount thereof to be dropped is in the range of 0.1 - 5 ml/drop and preferably in the range of 0.3 - 3 ml/drop, and the concentration of the aqueous solution of a polyvalent metal is in the range of 1 - 10 mass% and preferably in the range of 3 - 5 mass%. By fixing the concentration and the amount of dropping of the aqueous solution of sodium alginate and the concentration of the aqueous solution of a polyvalent metal salt within these ranges, it is made possible to prepare the hydrogel of a polyvalent metal salt of alginic acid possessing the shape, size and degree of crosslinking mentioned above.

The aspect ratio of the hydrogel to be obtained can be adjusted by the viscosity of the aqueous solution of sodium alginate to be used, the distance from the discharge port of the aqueous solution to the liquid level of the aqueous solution of the polyvalent metal salt, the shape of the discharge port (inside diameter 0.1 - 5 mm), etc. Specifically, the aspect ratio is small when the discharge outlet is in the shape of a buret, while the aspect ratio is large when the discharge outlet is in the shape of an identical inside diameter.

The adjustment of the degree of crosslinking is carried out, depending on the concentration of the polyvalent metal salt in the aqueous solution of a polyvalent metal salt and the duration of contact with the aqueous solution of the polyvalent metal salt. When an aqueous 0.5 mass% sodium alginate solution is dropped into an aqueous solution of a polyvalent metal salt and the resultant solution is washed with water, for example, the degree of corsslinking reaches 90% within one hour, the degree of crosslinking reaches 80% within 30 minutes, and the degree of crosslinking reaches30% within 5 minutes respectively of completing the addition.

The agent for preventing and healing constipation contemplated by this invention can be used for healing and preventing the symptoms of various sorts of constipation such as, for example, such habitual constipations as loose (pericolic) constipation, perirectal constipation, and convulsive constipation, transient simple constipation, and symptomatic constipation which are induced by various functional disorders and organic disorders of the defecating mechanism.

The agent for preventing and healing constipation according to this invention allows the aforementioned hydrogel of a polyvalent metal salt of alginic acid to be orally administered either alone or as incorporated in food or feed to human beings and animals. When the aforementioned hydrogel of a polyvalent metal salt of alginic acid is administered alone, this hydrogel of a polyvalent metal salt of alginic acid may be prepared for the use by properly incorporating therein such edulcorants as syrup, honey, sugar, salt, soy sauce, gravy, vanilla essence, and butter, taste enhancer, spice, flavoring agent, coloring agent, preservative, and antiseptic. When the aforementioned hydrogel of a polyvalent metal salt of alginic acid is added to food or feed, this hydrogel of a polyvalent metal salt of alginic acid or what is prepared by incorporating therein the aforementioned additives may be utilized as added to a varying kind of food such as water, milk, juice, lactic acid drink, yogurt, tea, miso soup, soup, boiled rice, rice cake, soybean curd, agar, jelly, pastry, or Japanese cake or to feed.

The dosage of the agent for preventing and healing constipation contemplated by this invention varies with the age, body weight, and symptom of the patient, the degree of gravity of the symptom, the effect of therapy, and the duration of treatment. Generally for adults, the dose of the hydrogel of a polyvalent metal salt of alginic acid is in the range of 5 - 80 g and preferably in the range of 10 - 60 g for the purpose of healing constipation or in the range of 5 - 30 g and preferably in the range of 10 - 20 g for the purpose of preventing constipation is given once to several times daily.

Since the agent for preventing and healing constipation contemplated by this invention is formed by containing the hydrogel of a polyvalent metal salt of alginic acid, it prevents the feces from hardening and consequently serves the purpose of preventing or healing constipation owing to the fact that the hydrogel of a polyvalent metal salt of alginic acid maintains the hydrated state against the action of water absorption in the bowel, particularly in the large intestine.

The submucosal plexus and the extrinsic parasympathetic nerves (vagus nerve and sacral nerve) which intervene between the colic mucous membrane and the smooth muscle layer are adjusting the motion and the defecating reflection of the large intestine. The hydrogel of a polyvalent metal salt of alginic acid manifests the function of promoting defecation because it reaches the interior of the large intestine without being digested while en route thereto and imparts moderate stimulation to the submucosal plexus in the large intestine.

Particularly the aforementioned hydrogelofa polyvalent metal salt of alginic acid in the agent for preventing and healing constipation as contemplated by this invention assumes a spherical or ovaloid shape having a short radius in the range of 1 - 10 mm and an aspect ratio (long diameter/short diameter) in the rnge of 1 - 2.5. It, therefore, moves easily in the feces and promotes the defecating reflecting by dint of the aforementioned stimulation of the submucosal plexus and brings a dramatic effect of preventing and healing constipation without entailing absolutely no secondary action.

Further, when the aforementioned hydrogel of a polyvalent metal salt of alginic acid having a degree of crosslinking in the range of 30 - 90% is used for the agent for preventing and healing constipation as contemplated by this invention, it acquires a structure containing the functional group (-COOH or -COONa) in an amount in the range of 10 - 70%, assumes an ability to maintain the aforementioned hydrated state powerfully, and manifests a more prominent effect of preventing and healing constipation.

The hydrogel according to this invention generally is stored in water or in a natural compound possessing a free acid group capable of reacting with and sequestering a basic compound such as indole, skatole, ammonia, hydrogen sulfide, and amines which are main components of fecal odor, i.e. hydrogel compounds such as, for example, alginic acid, pectin, polydextrose (which are carboxyl groups) and chondroitin sulfuric acid and carageenin (which are sulfuric acid groups) . The average molecular weight of such a natural compound is not less than 10,000 and preferably not less than 200,000. Though the ratio of the hydrogel of a polyvalent metal salt of alginic acid/water or the aforementioned natural compound does not need to be particularly restsricted, it is in the range of 2 : 1 - 1 : 2 and preferably in the range of 3 : 2 - 1 : 1 by volume.

Furthermore, by causing part or the whole of the hydrogel of the aforementioned natural compound in the agent for preventing and healing constipation contemplated by this invention to assume a structure containing activated carbon in an amount of 1 - 90 mass% and preferably 1 - 30 mass%, it is made possible to obtain an easily ingestible gel because the activated carbon adsorbs and eliminates the offensive odor such as the seaweed odor which is emitted while the hydrogel of a polyvalent metal salt of an alginic acid is undergoing a bactericidal treatment. The formation of this structure also manifests the effect of mending intestinal disorders by adsorbing and eliminating a toxic substance, a poisonous matter, and a harmful substance in the bowel, particularly in the large intestine and the effect of adsorbing and eliminating the fecal odor.

If the content of the activated carbon falls short of 1 mass%, the shortage will result in preventing the deodorization by the addition of activated carbon from being manifested fully satisfactorily. The activated carbon is ideally used in the form of a powder or particles having a particle diameter in the range of 100 - 1000 µm and preferably in the range of 200 - 300 µm. If the particle diameter of the activated carbon falls short of 100 µm, the shortage will be at a disadvantage in rendering the handling of the produced agent difficult. If the particle diameter exceeds 1000 µm, the overage will be at a disadvantage in impairing the palatability of the agent and degrading the ability of adsorption per unit weight of the active carbon.

Now, the agent for preventing and healing constipation contemplated by this invention will be described more specifically below with reference to working examples. This invention it not limited to these examples.

The degree of crosslinking was determined by the following method.

A calibration curve was produced by the infrared absorption spectrum according to the 2% potassium bromide tablet method using alginic acid and sodium salts thereof in varied ratios. This calibration curve was utilized for determining free acids. In the calibration of a given free acid, attention was focused on the characteristic absorption of the free acid, *v*_{c=0} 1710 cm⁻¹. The quantitative determination of a metal (calcium, etc.) was effected by chelate titration and gravimetric analysis. Elemental analysis was additionally utilized.

### Example 1

### Production of hydrogel of calcium alginate (1)

A sodium alginate solution was prepared by adding 10 g of sodium alginate kept in a stirred state piecemeal into 800 ml of water and diluting the resultant solution with distilled water to a total volume of 1000 ml. Separately, a calcium chloride solution was prepared by dissolving 30 g of calcium chloride in 800 ml of distilled water and diluting the resultant solution with distilled water to a total volume of 1000 ml.

Then, the aforementioned sodium alginate solution was injected into a 50-ml buret (inside diameter 0.55 mm) and dropped at a rate of 1 ml/drop into 500 ml of the aforementioned calcium chloride solution to obtain 50 g of a crude product of hydrogel of calcium alginate. This crude product was placed in a 5-liter vessel, left standing at rest for 0.4 hour at room temperature, and treated for 10 hours with running deionized water to expel an unaltered product and a by-product and obtain hydrogel of calcium alginate (1) having a short diameter in the range of 3.0 - 3.5 mm, an aspect ratio in the range of 1 - 1.1, and a degree of crosslinking of 70%. The hydrogel (1) was stored in water.

### Example 2

### Production of hydrogel of calcium alginate (2)

A hydrogel of calcium alginate (2) having a short diameter in the range of 3.0 - 3.2 mm, an aspect ratio in the range of 1 - 1.1, and a degree of crosslinking of 65% was obtained by substantially following the procedure of Example 1 while changing the concentration of calcium chloride to 2.5 mass% (providing the duration of standing at rest in the calcium chloride solution was 0. 35 hour) . The hydrogen (2) was stored in water.

### Example 3

### Production of hydrogel of calcium alginate (3)

A hydrogel of calcium alginate (3) having a short diameter in the range of 3.0 - 3.3 mm, an aspect ratio in the range of 1 - 1.05, and a degree of crosslinking of 60% was obtained by substantially following the procedure of Example 1 while changing the concentration of calcium chloride to 2.0 mass% (providing the duration of standing at rest in the calcium chloride solution was 0.3 hour). The hydrogen (3) was stored in water.

### Example 4

### Production of hydrogel of calcium alginate (4)

A hydrogel of calcium alginate (4) having a short diameter in the range of 2.8 - 3.0 mm, an aspect ratio in the range of 1 - 1.1, and a degree of crosslinking of 70% was obtained by substantially following the procedure of Example 1 while changing the concentration of calcium chloride to 1.5 mass% (providing the duration of standing at rest in the calcium chloride solution was 0.35 hour). The hydrogen (4) was stored in water.

### Example 5

### Production of hydrogel of calcium alginate (5)

A hydrogel of calcium alginate (5) having a short diameter in the range of 3.5 - 4.0 mm, an aspect ratio in the range of 1.1 - 1.2, and a degree of crosslinking of 65% was obtained by substantially following the procedure of Example 2 while changing the concentration of calcium chloride to 0.8 mass% (providing the duration of standing at rest in the calcium chloride solution was 0.3 hour) . The hydrogen (5) was stored in water.

### Example 6

### Production of activated carbon-containing hydrogel of calcium alginate

A thorough mixture of 10 g of sodium alginate and 50 g of activated carbon having an average particle diameter of 250 µm was added in a stirred state piecemeal into 800 ml of water. The resultant solution was diluted with water to a total volume of 1000 ml and stirred for 24 hours to prepare a sodium alginate solution containing 5 mass% of activated carbon. Separately, a calcium chloride solution was prepared by dissolving 30 g of calcium chloride in 800 ml of distilled water and diluting the resultant solution with distilled water to a total volume of 1000 ml.

Then, the aforementioned activated carbon-containing sodium alginate solution was injected into a 50-ml buret and added at a rate of 2 ml/drop into 500 ml of the aforementioned calcium chloride solution to obtain 3.5 g of a crude product of activated carbon-containing hydrogel of calcium alginate. This crude product was placed in a 5-liter vessel, left standing at rest for 0.4 hour at room temperature, and treated for 10 hours with running deionized water to expel unaltered product and by-product and obtain an activated carbon-containing hydrogel of calcium alginate (6) having a short diameter in the range of 3 - 3.5 mm, an aspect ratio in the range of 1 - 1.1, and a degree of crollinking of 70%. This hydrogel was stored in water.

### Example 7

### Production of hydrogel of calcium alginate (6)

A hydrogel of calcium alginate (6) having a short diameter in the range of 3.6 - 4.0 mm, an aspect ratio in the range of 1.1-1.5, and a degree of crosslinking of 70% was obtained by substantially following the procedure of Example 1 while changing the concentration of sodium alginate to 2 mass% and forming the leading terminal part of the outlet port in an identical inside diameter type having an inside diameter of 3 mm (providing that the duration of standing at rest in the calcium chloride solution was 0.6 hour). This hydrogel was stored in water.

### Example 8

### Test for confirming effect of preventing and healing constipation (1)

A female 21 years old having no motion over a period of 10 days was made to ingest miso soup containing 60 ml of the hydrogel of calcium alginate (1) obtained in Example 1 during every meal by way of oral administration. In consequence of four administrations, the female had thorough defecation within 30 minutes of taking the last meal. When the administration of 20 ml of the hydrogel of calcium alginate (1) was similarly continued for one month, the female had normal defecation once every day during this period without experiencing any discernible sign of special secondary action.

### Example 9

### Test for confirming effect of preventing and healing constipation (2)

A female 25 years old having no motion over a period of 7 days was made to ingest miso soup containing 50 ml of the hydrogel of calcium alginate (2) obtained in Example 2 during every breakfast by way of oral administration. In consequence of five administrations, the female had thorough defecation within 30 minutes of taking the last meal. When the administration of 20 ml of the hydrogel of calcium alginate (2) was similarly continued for one month, the female had normal defecation once every day during this period without experiencing any discernible sign of special secondary action.

### Example 10

### Test for confirming effect of preventing and healing constipation (3)

A male 40 years old having no motion over a period of 4 days was made to ingest miso soup or milk containing 60 ml of the hydrogel of calcium alginate (3) obtained in Example 3 during every meal by way of oral administration. In consequence of four administrations, the male had thorough defecation within 60 minutes of taking the last meal. When the administration of 20 ml of the hydrogel of calcium alginate (3) was similarly continued for one month, the male had normal defecation once every day during this period without experiencing any discernible sign of special secondary action.

### Example 11

### Test for confirming effect of preventing and healing constipation (4)

A female 18 years old having no motion over a period of 10 days was made to ingest agar gel containing 50 ml of the hydrogel of calcium alginate (4) obtained in Example 4 during every breakfast with the object of orally administering the hydrogel of calcium alginate (4). In consequence of six administrations, the female had thorough defecation within 30 minutes of taking the last meal. When the administration of 30 ml of the hydrogel of calcium alginate (4) was similarly continued for three months, the female had normal defecation once every day during this period without experiencing any discernible sign of special secondary action.

### Example 12

Test for confirming effect of preventing and healing constipation (5)

A female 32 years old having no motion over a period of 5 days was made to ingest soybean curd containing 50 ml of the hydrogel of calcium alginate (5) obtained in Example 5 during every breakfast with the object of orally administering the hydrogel of calcium alginate (5). In consequence of four administrations, the female had thorough defecation within 60 minutes of taking the last meal. When the administration of 30 ml of the hydrogel of calcium alginate (5) was similarly continued for one month, the female had normal defecation once every day during this period without experiencing any discernible sign of special secondary action.

### Example 13

### Test for confirming effect of preventing and healing constipation (6)

A female 23 years old having no motion over a period of 7 days was made to ingest miso soup containing 50 ml of a mixture resulting from mixing the hydrogel of calcium alginate (1) obtained in Example 1 and the activated carbon-containing hydrogel of calcium alginate (6) obtained in Example 6 at a ratio of 6 : 1 during every breakfast by way of oral administration. In consequence of four administrations, the female had thorough defecation within 30 minutes of taking the last meal. When the administration of 20 ml of the aforementioned mixture was similarly continued for one month, the female had normal defecation once every day during this period without experiencing any discernible sign of special secondary action.

### Example 14

### Test for confirming effect of preventing and healing constipation (7)

A male 30 years old having motion at intervals of one to two days but always complaining of hardness of feces and occasionally suffering the anus to tear and bleed was made to ingest miso soup containing 30 ml of the activated carbon-containing hydrogel of calcium alginate (6) obtained in Example 6 during every meal for two months by way of oral administration. On the second day of the administration onward, the male was enabled to discharge feces of normal hardness. Thereafter, the male had normal defecation once daily without experiencing any discernible sign of special secondary action.

### Example 15

### Test for confirming effect of preventing and healing constipation (8)

A female 18 years old having no motion over a period of 10 days was made to ingest milk containing 50 ml of the hydrogel of calcium alginate (6) obtained in Example 6 during every breakfast with the object of orally administering the hydrogel of calcium alginate (6). In consequence of three administrations, the female had thorough defecation within 120 minutes of taking the last meal. When the administration of 30 ml of the hydrogel of calcium alginate (6) was similarly continued for one month, the female had normal defecation once every day during this period without experiencing any discernible sign of special secondary action.

### Example 16

### Test for confirming effect of preventing and healing constipation (9)

A female 25 years old having no motion over a period of 7 days was made to ingest 50 g of what was obtained by thoroughly mixing 50 g of the hydrogel of calcium alginate (1) obtained in Example 1 and 50 g of water swelled alginic acid during each of the three meals daily. From the third ingestion onward, the female had normal defecation and sharp decrease of fecal odor.

### Example 17

### Test for confirming effect of preventing and healing constipation (10)

A female 30 years old having no motion over a period of 7 days was made to ingest 100 g of what was obtained by thoroughly mixing 150 g of the hydrogel of calcium alginate (1) obtained in Example 1 and 150 g of 0.5% carageenin gel during each of the three meals daily. From the fourth ingestion onward, the female had normal defecation and experienced virtually no discernible fecal odor.

### Example 18

### Test for confirming effect of prevention and healing constipation (11)

A male 40 years old having no motion over a period of 10 days was made to ingest 100 g of what was obtained by thoroughly mixing 150 g of the hydrogel of calcium alginate (6) obtained in Example 6 and 150 g of 3% pectin gel during each of the three meals daily. From the second ingestion onward, the male had normal defecation and experienced virtually no discernible fecal odor.

### Comparative Example 1

### Test for confirming effect of preventing and healing constipation (12)

When a female 24 years old having no motion over a period of four days was made to ingest 50 ml of konjak gel during every breakfast for 10 days, the female was not relieved of constipation.

### Comparative Example 2

### Test for confirming effect of preventing and healing constipation (13)

When a male 35 years old having irregular defecation at intervals of one to three days but always complaining hardness of feces was made to ingest 60 ml of agar gel during every meal for one month, the male failed to enjoy complete heal of the symptom of constipation.

## Claims

1. An agent for preventing and healing constipation, containing a hydrogel of a polyvalent metal salt of alginic acid possessing a spherical or ovaloid shape having a short diameter in the range of 1 - 10 mm and an aspect ratio (long diameter/short diameter) in the range of 1 - 2.5.

2. An agent according to claim 1, wherein said hydrogel of a polyvalent metal salt of alginic acid is obtained by dropping an aqueous solution of sodium alginate into an aqueous solution of a polyvalent metal salt.

3. An agent according to claim 1 or claim 2, wherein said hydrogel of a polyvalent metal salt of alginic acid has a degree of crosslinking in the range of 30 - 90%.

4. An agent according to any of claims 1 - 3, wherein said polyvalent metal salt of alginic acid is calcium alginate.

5. An agent according to any of claims 1 - 3, wherein the particles of said polyvalent metal salt of alginic acid are stored in water and/or a hydrogel compound of a natural compound possessing a free acid group capable of reacting with and sequestering a basic compound.

6. An agent according to claim 5, wherein the molecular weight of said natural compound is not less than 10,000.

7. An agent according to claim 6, wherein said acid group is carboxyl group or sulfuric acid group.

8. An agent according to claim 5, wherein said natural compound is at least one member selected from the group consisting of alginic acid, pectin, polydextrose, chondroitin sulfuric acid, and carageenin.

9. An agent according to any of claims 5 - 8, wherein the hydrogel of said natural compound contains an activated carbon in an amount in the range of 1 - 90 mass%.

10. An agent according to any of claims 5 - 9, wherein the volume ratio of said hydrogel of a polyvalent metal salt of alginic acid/water or said natural substance is in the range of 2 : 1 - 1 : 2.

11. A method for the production of an agent for preventing and healing constipation containing a hydrogel of a polyvalent metal salt of alginic acid, comprising the steps of dropping an aqueous solution of sodium alginate into an aqueous solution of a polyvalent metal salt and subsequently washing the resultant solution with water.

12. A method according to claim 11, wherein the concentration of the aqueous solution of sodium alginate is in the range of 0 . 002 - 5 mass% and the concentration of the polyvalent metal salt is in the range of 1 - 10 mass%.

13. A method according to claim 11 or claim 12, wherein the amount of the aqueous solution of sodium alginate to be dropped is in the range of 0.1 - 5 ml/drop.

14. A method according to any of claims 11 - 13, wherein said aqueous solution of sodium alginate is dropped through a nozzle having the inside diameter of the leading terminal part thereof in the range of 0.1 - 5 mm.
